# EUROPÄISCHE PATENTANMELDUNG

(11) **EP 2 347 750 A1**
(43) Veröffentlichungstag der Anmeldung: **27.07.2011**
(21) Anmeldenummer: 10151718.3
(22) Anmeldetag: 26.01.2010
(51) Int. Cl.: A61J 1/20

(54) **Konnektor für medizinischen Wirkstoff enthaltende Behälter**

(71) Anmelder: Fresenius Kabi Deutschland GmbH, 61352 Bad Homburg (DE)
(72) Erfinder: Rahimy, Ismael, 61169, Friedberg (DE); Brandenburger, Torsten, 61203, Reichelsheim (DE)
(74) Vertreter: Fresenius Kabi Deutschland GmbH

(57) **Zusammenfassung**

Der erfindungsgemäße Konnektor (1, 1') umfasst einen ersten Anschlussbereich (3a, 3a') für den Anschluss eines ersten Behälters (2), ein Durchstechelement (4, 4') und ein Anbrechelement (5), wobei das Durchstechelement (4, 4') einen ersten Endabschnitt (6a, 6a') und einen zweiten Endabschnitt (6b, 6b') und einen vom ersten Endabschnitt (6a, 6a') zum zweiten Endabschnitt (6b, 6b') verlaufenden Kanal (7) für den Transport eines medizinischen Wirkstoffs aufweist, wobei der erste Endabschnitt (6a, 6a') zum Durchstechen einer Membran (8) des im ersten Anschlussbereich angeschlossenen ersten Behälters (2) ausgebildet ist, und am zweiten Endabschnitt (6b, 6b') das Anbrechelement (5) anbrechbar und/oder abbrechbar befestigt ist, wobei im befestigten Zustand das Anbrechelement (5) den Kanal (7) des Durchstechelements (4, 4') flüssigkeitsdicht verschließt.

## Beschreibung

### Technisches Gebiet

Die Erfindung betrifft einen Konnektor für medizinischen Wirkstoff enthaltende Behälter, der es ermöglicht, Wirkstoff aus einem Behälter in einen anderen Behälter zu überführen.

### Stand der Technik

Die EP 1 066 812 A2 offenbart einen Konnektor für medizinischen Wirkstoff enthaltende Behälter, der einen ersten Anschlussbereich für den Anschluss eines Glasfläschchens, einen zweiten Anschlussbereich für den Anschluss eines flexiblen Beutels und ein Durchstechelement aufweist. Das Durchstechelement ist in Form einer zweiseitigen Kanüle ausgebildet. Der erste und der zweite Anschlussbereich werden durch ein gemeinsames Gehäuse ausgebildet. Das Gehäuse ist im zweiten Anschlussbereich mit einem Verbindungsmittel lösbar verbindbar, wobei das Verbindungsmittel mit dem flexiblen Beutel verbunden ist. Das Verbindungsmittel umfasst eine Membran, die den Zugang zum Beutel flüssigkeitsdicht verschließt. Durch Anschließen des Glasfläschchens an das Gehäuse im ersten Anschlussbereich durchdringt das Durchstechelement einen im Deckel des Glasfläschchens vorgesehenen Gummistopfen. Durch Anschließen des Verbindungsmittels an das Gehäuse des Konnektors durchdringt das Durchstechelement auch die im Verbindungsmittel angeordnete Membran. Durch das Durchdringen der Membran sowohl des Glasfläschchens als auch der Membran des Verbindungsmittels kann durch das Durchstechelement ein sich in einem der Behälter befindender Wirkstoff in den anderen Behälter überführt werden.

### Kurzbeschreibung der Erfindung

Der erfindungsgemäße Konnektor für medizinischen Wirkstoff enthaltende Behälter umfasst einen ersten Anschlussbereich für den Anschluss eines ersten Behälters, ein Durchstechelement und ein Anbrechelement, wobei das Durchstechelement einen ersten Endabschnitt und einen zweiten Endabschnitt und einen vom ersten Endabschnitt zum zweiten Endabschnitt verlaufenden Kanal für den Transport eines medizinischen Wirkstoffs aufweist, wobei der erste Endabschnitt zum Durchstechen einer Membran eines im ersten Anschlussbereich angeschlossenen ersten Behälters ausgebildet ist, und am zweiten Endabschnitt das Anbrechelement anbrechbar und/oder abbrechbar befestigt ist, wobei im befestigten Zustand das Anbrechelement den Kanal des Durchstechelements flüssigkeitsdicht verschließt.

Das Anbrechelement hält im befestigten Zustand den Kanal des Durchstechelementes verschlossen, so dass aus einem am ersten Anschlussbereich des Konnektors vollständig angeschlossenen Behälter noch kein Wirkstoff aus dem Behälter entnommen oder in diesen überführt werden kann. Erst durch das Anbrechen und/oder Abbrechen des Anbrechelementes ist ein Transport des Wirkstoffes durch das Durchstechelement ermöglicht. Das Anbrechen und/oder Abbrechen des Anbrechelementes ermöglicht ein schnelles, sicheres und steriles Öffnen des Kanals von Außen ohne die Gefahr von Verletzungen oder Beschädigungen des Benutzers oder des anzuschließenden Behälters, im Gegensatz beispielsweise zum Durchstechen einer Membran mit einer Kanüle. Durch die Befestigung des Anbrechelementes am Endabschnitt des Durchstechelementes ist der Konnektor kompakt ausführbar und es kann auf zusätzliche Elemente, die das Anbrechelement in Position halten, verzichtet werden.

Die Behälter, die durch den Konnektor verbunden oder angeschlossen werden können, können sowohl geschlossene Behälter, beispielsweise Glasflaschen, Kunststoffflaschen oder Beutel, insbesondere flexible Beutel, oder ,"offene" Behälter, beispielsweise Katheter oder sonstige Leitungen, sein, wie sie beispielsweise in der Infusion, Transfusion, der klinischen Ernährung, Onkologie, Dialyse oder anderen medizinischen Feldern verwendet werden. Der medizinische Wirkstoff, der mittels des Konnektors von einem Behälter zum anderen Behälter übertragbar ist, kann beispielsweise eine Flüssigkeit oder ein Pulver sein.

Die Sollbruchstelle, an der das Anbrechteil abgebrochen wird, ist vorzugsweise derart ausgebildet, dass das Anbrechteil durch eine Anbrechbewegung lediglich teilweise abgebrochen wird, also angebrochen wird, so dass der Wirkstoff zwar durch den Kanal hindurchtreten kann, aber das Anbrechteil mit dem Durchstechelement verbunden bleibt und somit nicht in einen Behälter gelangen kann. Des Weiteren ist der Konnektor derart ausgebildet, dass das Anbrechen des Anbrechteils vorzugsweise einhändig mit Daumen und Zeigefinger erfolgen kann. In einer alternativen Variante ist die Sollbruchstelle für ein vollständiges Abbrechen des Anbrechelementes ausgebildet. Das vollständige Abbrechen des Anbrechelementes kann auch nach einem Anbrechen erfolgen.

In einer vorteilhaften Ausführungsform der Erfindung sind das Durchstechelement und das Anbrechelement einteilig ausgebildet, vorzugsweise als Spritzgussteil. Dies ermöglicht eine kostengünstige Herstellung des Konnektors. Alternativ wäre es beispielsweise ebenfalls möglich, Durchstechelement und Anbrechelement mehrteilig auszubilden und das Anbrechelement mit dem Durchstechelement flüssigkeitsdicht zu verbinden, beispielsweise mittels eine Stoffschlusses oder anderen geeigneter Verbindungstechniken, beispielsweise Schweißen oder Kleben.

In einer weiteren vorteilhaften Ausführungsform umfasst der Konnektor ein Gehäuse mit einem den ersten Anschlussbereich ausbildenden ersten Gehäuseabschnitt und einem Gehäuseboden, wobei das Durchstechelement durch den Gehäuseboden hindurchtritt.

Vorzugsweise sind Gehäuse, Durchstechelement und Anbrechelement einteilig ausgebildet, beispielsweise als Spritzgussteil. Des Weiteren ist es bevorzugt, Durchstechelement, Anbrechelement und gegebenenfalls Gehäuse aus einem Kunststoff herzustellen, insbesondere aus einem Polypropylen, Polycarbonat und/oder einem Blend, vorzugsweise ein Blend aus Polypropylen und Styrol/Ethylen/Butylen/Styrol-Block-Copolymerisat (SEBS).

Weitere vorteilhafte Ausführungsformen sind in den abhängigen Ansprüchen beschrieben.

Die Erfindung wird im Folgenden anhand von Ausführungsbeispielen, die durch mehrere Figuren dargestellt sind, näher beschrieben.

### Kurze Beschreibung der Zeichnungsfiguren

Es zeigt:

Fig. 1 einen Längsschnitt durch eine erste Ausführungsform eines erfindungsgemäßen Konnektors,

Fig. 2 eine erste perspektivische Ansicht des in Fig. 1 gezeigten Konnektors,

Fig. 3 eine weitere perspektivische Ansicht des in Fig. 1 gezeigten Konnektors,

Fig. 4 eine perspektivische Ansicht des Verbindungsmittels des in Fig. 1 gezeigten Konnektors,

Fig. 5 eine Ansicht des in Längsachse aufgeschnittenen Konnektors mit einer in einer Andockposition angeschlossenen Glasflasche,

Fig. 6 eine Ansicht des in Längsachse aufgeschnittenen Konnektors mit einer in einer Endposition angeschlossenen Glasflasche,

Fig. 7 einen Längsschnitt durch eine zweite Ausführungsform eines erfindungsgemäßen Konnektors mit Luer-Lock-Verbindung,

Fig. 8 eine erste perspektivische Ansicht des in Fig. 7 gezeigten Konnektors ,

Fig. 9 eine weitere perspektivische Ansicht des in Fig. 7 gezeigten Konnektors,

Fig. 10 eine Ansicht des in Längsachse aufgeschnittenen, in Fig. 7 gezeigten Konnektors.

### Beschreibung der Ausführungsarten

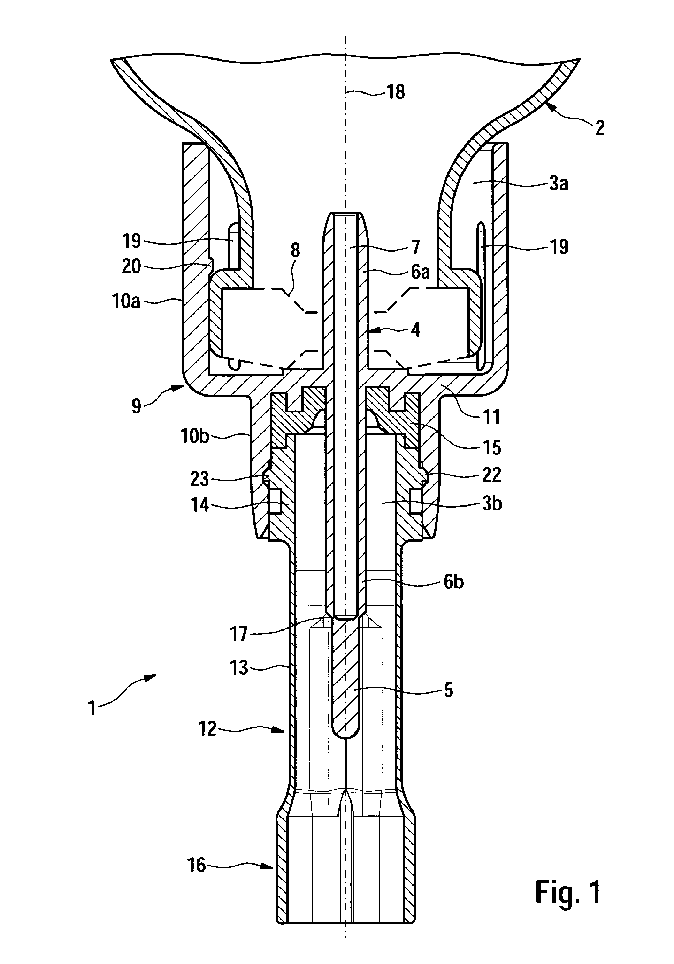

Die Figuren 1 bis 6 zeigen in verschiedenen Ansichten eine erste Ausführungsform eines erfindungsgemäßen Konnektors 1 für medizinischen Wirkstoff enthaltende Behälter.

Der Konnektor 1 umfasst einen ersten Anschlussbereich 3a für den Anschluss eines ersten Behälters, einen zweiten Anschlussbereich 3b für den Anschluss eines zweiten Behälters, ein Durchstechelement 4 und ein Anbrechelement 5. Des Weiteren umfasst der Konnektor 1 ein Gehäuse 9 mit einem ersten Gehäuseabschnitt 10a, einem zweiten Gehäuseabschnitt 10b und einem Gehäuseboden 11, wobei der erste Gehäuseabschnitt 10a den ersten Anschlussbereich 3a und der zweite Gehäuseabschnitt 10b den zweiten Anschlussbereich 3b ausbildet. Das Durchstechelement 4 tritt durch den Gehäuseboden 11 hindurch. Das Gehäuse 9, das Durchstechelement 4 und das Anbrechelement 5 sind im Wesentlichen rotationssymmetrisch um eine gemeinsame Symmetrieachse 18 ausgebildet.

Das Durchstechelement 4 ist in Form eines Röhrchens ausgebildet. Das Durchstechelement 4 umfasst einen ersten Endabschnitt 6a, der hier in den Anschlussbereich 3a hineinragt, und einen zweiten Endabschnitt 6b, der hier über dem zweiten Anschlussbereich 3b liegt. Der innere Hohlraum des Durchstechelementes 4 bildet einen vom ersten Endabschnitt 6a zum zweiten Endabschnitt 6b verlaufenden Kanal 7 für den Transport eines medizinischen Wirkstoffs. Der erste Endabschnitt 6a ist zum Durchstechen einer Membran eines im ersten Anschlussbereich 3a angeschlossenen Behälters ausgebildet, beispielsweise zum Durchstechen eines Gummistopfens 8 (durch eine gestrichelte Linie angedeutet) eines Glasfläschchens 2. Zum Erleichtern des Durchstechens ist das Ende des Endabschnitts 6a mit einer scharfen Spitze ausgebildet, vorzugsweise angeschrägt oder angespitzt, beispielsweise in Form einer Kanüle. Am zweiten Endabschnitt 6b ist das Anbrechelement 5 unmittelbar an der Spitze des Durchstechelementes 4 manuell anbrechbar befestigt, wobei im befestigten Zustand das Anbrechelement 5 den Kanal 7 des Durchstechelementes 4 flüssigkeitsdicht verschließt. Eine Verbindungsstelle 17 zwischen Anbrechelement 5 und Durchstechelement 4 ist derart ausgebildet, dass durch Abknicken des Anbrechelementes 5 gegenüber dem Durchstechelement 4 der Kanal 7 geöffnet wird, aber die Verbindung mit des Anbrechelementes 5 mit dem Durchstechelement 4 teilweise bestehen bleibt, so dass das Anbrechelement 5 sich nicht vom Durchstechelement 4 löst. In diesem Ausführungsbeispiel ist die Verbindungsstelle 17 als radial umlaufender Steg ausgebildet, der die Spitze des Endabschnitts 6b mit dem gegenüberliegenden Ende des Anbrechelementes 5 verbindet. Das Anbrechelement 5 ist in Form eines Stifts ausgebildet, der sich im befestigten Zustand axial und hinterschnittsfrei an das Durchstechelement 4 anschließt.

Alternativ kann die Verbindungsstelle 17 derart ausgebildet sein, dass das Anbrechelement 5 durch eine Abknickbewegung vollständig abbrechbar ist. In einer weiteren Variante kann ein Abbrechen des Anbrechelementes 5 durch ein Anbrechen und ein darauf folgendes Abbrechen erfolgen, beispielsweise durch zwei entgegengesetzte Biegebewegungen.

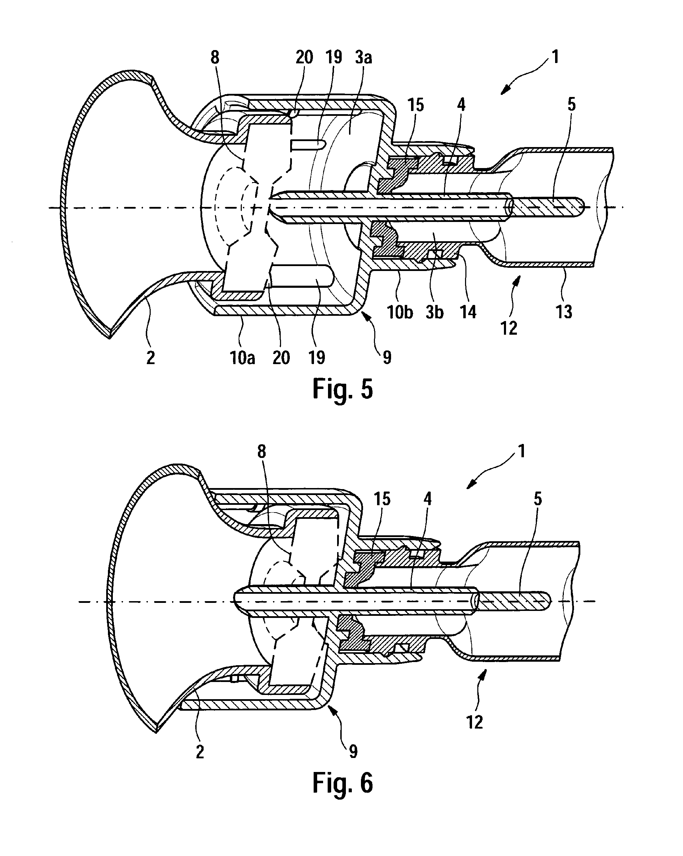

Der erste Gehäuseabschnitt 10a und der zweite Gehäuseabschnitt 10b sind im Wesentlichen hohlzylinder- oder rohrförmig ausgebildet. Der Außenradius des ersten Gehäuseabschnittes 10a, der hier für die Aufnahme eines Glasfläschchens 2 ausgebildet ist, ist größer als der Außenradius des zweiten Gehäuseabschnittes 10b. Der Gehäuseboden 11 liegt zwischen dem ersten Gehäuseabschnitt 10a und dem zweiten Gehäuseabschnitt 10b und bildet auf der jeweiligen Seite den Boden des ersten Anschlussbereiches 3a und des zweiten Anschlussbereiches 3b. Der erste Gehäuseabschnitt 10a umfasst mehrere axial verlaufende Klemmstege 19, die den Zweck haben, beim Einführen des Behälters 2 in den Anschlussbereich 3a diesen schon in einer Andockposition, in der das Durchstechelement 4 die Membran 8 noch nicht durchstoßen hat (siehe Figur 5), durch Klemmung vorzufixieren und insbesondere ein unerwünschtes Herausrutschen des Behälters, beispielsweise durch die Schwerkraft, zu verhindern. Auf den Klemmstegen 19 sind auf einer gemeinsamen Höhe Vorsprünge 20 vorgesehen, die einen Hinterschnitt bilden, die den Zweck haben, in einem vollständig eingeführten Zustand des Behälters 2, bei dem das Durchstechelement 4 die Membran 8 durchdrungen hat (siehe auch Figur 6), den Behälter 2 durch Formschluss zusätzlich festzuhalten.

Im Anschlussbereich 3a kann ein weiterer Hinterschnitt vorgesehen sein, beispielsweise durch zusätzliche Vorsprünge, der den Behälter 2 bereits in der Andockposition unter Ausbildung eines Formschlusses fixiert. Die Klemmstege 19 können in dieser Variante ebenfalls vorhanden sein, alternativ kann auf diese aber auch verzichtet werden. Ein solcher zweiter Hinterschnitt erleichtert das Verbinden von Behältern mit unterschiedlichen Abmaßen mit dem Konnektor 1.

Gehäuse 9, Durchstechelement 4 und Anbrechelement 5 sind in diesem Ausführungsbeispiel einteilig als Spritzgussteil ausgebildet. Als Material bevorzugt ist ein thermoplastische Kunststoff, vorzugsweise ein Polypropylen, Polycarbonat und/oder ein Blend, vorzugsweise ein Blend aus Polypropylen und Styrol/Ethylen/Butylen/Styrol-Block-Copolymerisat. Des Weiteren ist es bevorzugt, einen harten Kunststoff einzusetzen. Dies gewährleistet eine geeignete Steifigkeit bzw. ein reproduzierbares Abbrechverhalten dieses Teils.

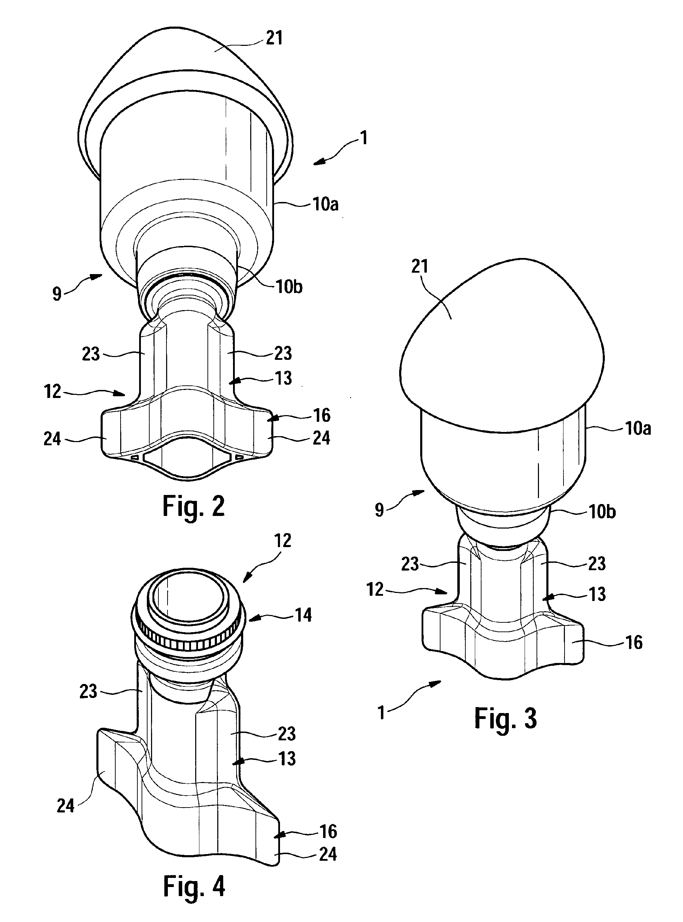

Der Konnektor 1 weist in diesem Ausführungsbeispiel zusätzlich eine manuell abziehbare Abdeckfolie 21 auf (siehe Fig. 2 und Fig. 3), beispielsweise eine Aluminium- oder Kunststofffolie, die den ersten Anschlussbereich 3a zum Schutz des ersten Anschlussbereichs 3a gegen Verunreinigung abdeckt. Eine derartige Abdeckfolie 21 ist optional, der Konnektor 1 kann ohne diese ausgebildet sein.

Der Konnektor 1 umfasst des Weiteren ein zum Transport des medizinischen Wirkstoffs ausgebildetes Verbindungsmittel 12. Das Verbindungsmittel 12 umfasst einen manuell elastisch verformbaren Biegeabschnitt 13, einen Verbindungsabschnitt 14 und einen Schweißabschnitt 16. Das Verbindungsmittel 12 ist über den Verbindungsabschnitt 14 im zweiten Anschlussbereich 3b an dem zweiten Gehäuseabschnitt 10b mit dem Gehäuse 9 einschnappend verbunden. Das Verbindungsmittel 12 ist als Hohlkörper ausgebildet und umgibt den zweiten Endabschnitt 6b und das Anbrechelement 5. Das Anbrechelement 5 befindet sich zumindest teilweise in dem elastisch verformbaren Biegeabschnitt 13, so dass durch einmaliges Knicken des Biegeabschnittes 13 das Abrechelement 5 anbrechbar ist.

Der Verbindungsabschnitt 14 umfasst einen an der Außenfläche radial umlaufenden Befestigungssteg 22, der in eine dazu korrespondierende Nut 23 an der Innenfläche des zweiten Gehäuseabschnitts 10b durch Einstecken des Verbindungsabschnittes 14 in den zweiten Gehäuseabschnitt 10b eingerastet ist. Durch diesen so gebildeten Formschluss ist das Verbindungsmittel 12 an dem Gehäuse 9 fixiert. Der Konnektor 1 umfasst ein Dichtungsmittel 15, hier eine Dichtungsmembran aus Silikon, einem thermoplastischen Elastomer (TPE) oder Polyisopren, die am Boden des zweiten Anschlussbereiches 3b zwischen Gehäuse 9 und Verbindungsmittel 12 angeordnet ist und das Verbindungsmittel 12 flüssigkeitsdicht gegen das Gehäuse 9 abdichtet, so dass bei einem Transport eines medizinischen Wirkstoffs durch das Durchstechelement 4 und das Verbindungsmittel 12 der Wirkstoff nicht im Verbindungsbereich zwischen Gehäuse 9 und Verbindungsmittel 12 austreten kann. Durch das Dichtungsmittel 15 ist gewährleistet, dass auch bei der Verwendung von unterschiedlichen Materialien für Verbindungsmittel 12 und Gehäuse 9 eine ausreichende Dichtheit vorliegt, auch im Falle von beispielsweise Temperaturschwankungen. Des Weiteren können auch Toleranzschwankungen berücksichtigt werden. Das Dichtungsmittel 15 ist zwischen dem Verbindungsmittel 12 und dem Gehäuse 9 unter elastischer Verformung des Dichtmittels 15 klemmend gehalten.

Der Biegeabschnitt 13 umfasst zwei außenseitige, axial verlaufende und spiegelsymmetrisch ausgebildete Versteifungsstege 23, siehe Figs. 2, 3. Die Versteifungsstege 23 bewirken einerseits eine Versteifung des Verbindungsmittels 12 im Biegeabschnitt 13, andererseits legen die Versteifungsstege 23 eine definierte Knickrichtung fest, in der das Verbindungsmittel 12 im Biegeabschnitt 13 abknickbar und damit das Anbrechelement 5 anbrechbar ist.

Der Schweißabschnitt 16 umfasst zwei flügelförmige, spiegelsymmetrisch ausgebildete Schweißlaschen 24, die hier als Fortsetzung der Versteifungsstege 23 ausgebildet sind. Die Schweißlaschen eignen sich insbesondere für das flüssigkeitsdichte Verbinden des Verbindungsmittels 12 mit einem hier nicht näher dargestellten flexiblen Beutel.

Das Verbindungsmittel 12 ist einteilig aus einem Kunststoff ausgebildet. Bevorzugt ist ein relativ zum Material des Gehäuses 9 flexibler Kunststoff, vorzugsweise ein mit dem Material des jeweiligen Behältnisses, hier des Beutels, verschweißbarer thermoplastischer Kunststoff, beispielsweise ein Polypropylen oder ein Polypropylen-SEBS-Blend. Ein flexibler Kunststoff ermöglicht die Ausbildung des manuell elastisch verformbaren Biegeabschnitts 13. Verbindungsabschnitt 14 und Schweißabschnitt 16 weisen eine im Vergleich zum Biegeabschnitt 13 dickere Wandstärke und/oder Versteifungen auf, wodurch diese Abschnitte relativ formsteif sind, was vorteilhaft für eine sichere Verbindung des Verbindungsmittels 12 mit dem Gehäuse 9 und dem Beutel oder einem anderen Behältnis ist.

Zum Anschließen eines Behältnisses an den Konnektor 1 wird zunächst die Abdeckfolie 21 entfernt. Anschließend wird das Behältnis, beispielsweise der als Glasfläschchen ausgebildete Behälter 2, in den ersten Anschlussbereich 3a eingeführt, bis dieser auf die Vorsprünge 20 trifft ("Andockposition"), siehe Fig. 2. In dieser Stellung ist der Behälter 2 vorfixiert, aber die Membran 8 des Behälters 2 noch nicht durchstoßen, so dass der Behälter 2 ohne Öffnen des Behälters 2 vom Konnektor 1 wieder trennbar ist. Beim weiteren Einführen des Behälters 2 wird die Membran 8 durch das Durchstechelement 4 durchstoßen, der Behälter 2 wird durch die Vorsprünge 20 formschlüssig am Konnektor 1 fixiert ("Endposition"), siehe Fig. 6. Durch Biegen/Knicken des Verbindungsmittels 12 im Biegeabschnitt 13 kann nun das Anbrechelement 5 angebrochen und damit der Kanal 7 geöffnet werden, wodurch ein Transport eines Wirkstoffes, beispielsweise einer Flüssigkeit, zwischen einem mit dem Verbindungsmittel 12 verbundenen Behältnis oder Mischen und Auflösen eines Pulvers mit dem Inhalt eines mit dem Verbindungsmittel 12 verbundenen Behältnisses, insbesondere einem flexiblen Beutel, und dem Behälter 2 möglich wird. Das Biegen oder Knicken des Verbindungsmittels 12 kann insbesondere einhändig durch Einsatz von Daumen und Zeigefinger erfolgen.

Der Behälter 2 kann in der Andockposition am Konnektor 1 vormontiert sein. Behälter 2, Konnektor 1 und ein mit dem Konnektor 1 verbundenes zweite Behältnis, insbesondere ein Beutel, können in einem Umbeutel als Set zusätzlich eingeschlossen sein. Dies ermöglicht das schnelle Mischen, Überführen, Auflösen oder Verdünnen eines Wirkstoffes außerhalb des Laminar-Flow-Bereiches, ohne das die Gefahr besteht, dass Wirkstoff ungewünscht nach außen tritt oder kontaminiert wird.

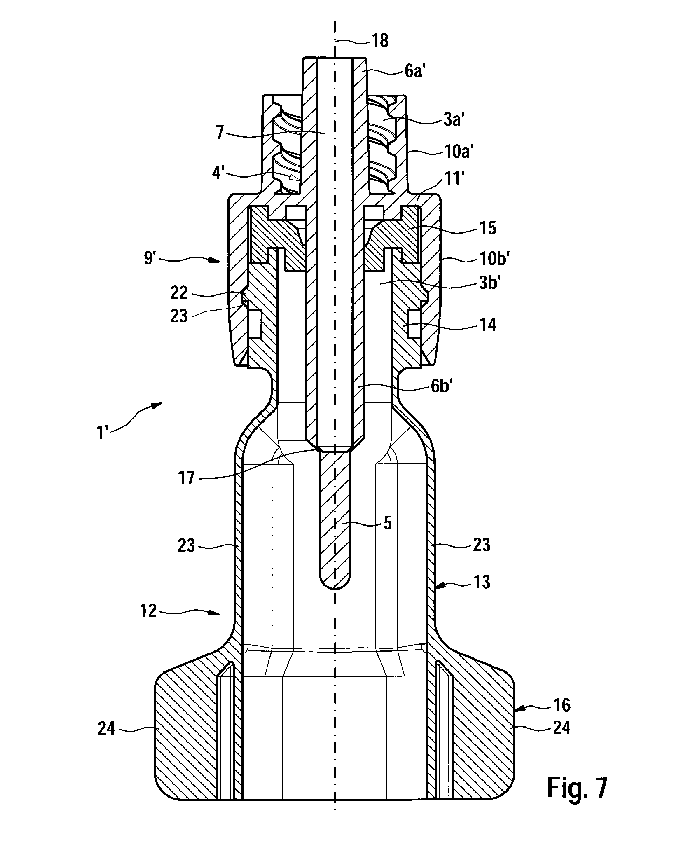

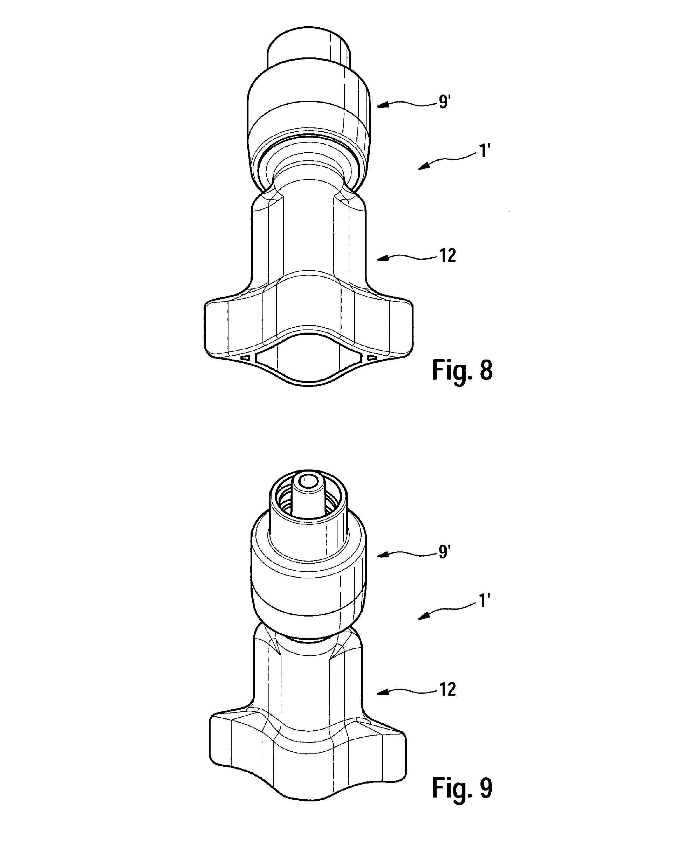

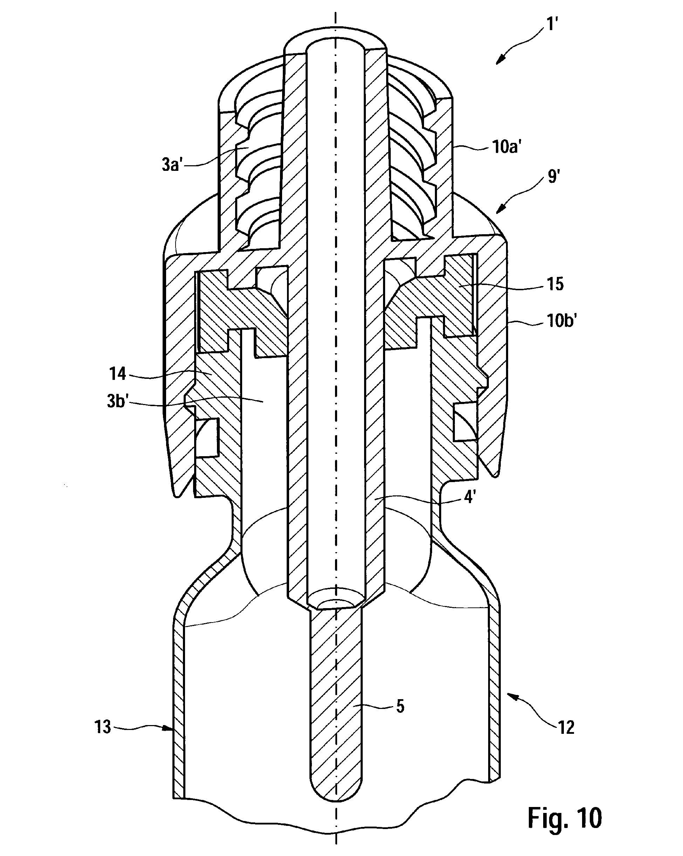

Die Figuren 7 bis 10 zeigen eine zweite Ausführungsform eines erfindungsgemäßen Konnektors 1'. '.

Gemäß der zweiten Ausführungsform ist der erste Anschlussbereich 3a' und dementsprechend der erste Gehäuseabschnitt 10a' und der Gehäuseboden 11' als Male-Teil eines Luer-Locks (ISO 594/1-1986) ausgebildet. Das Verbindungsmittel 12, das Dichtmittel 15 und der zweite Gehäuseabschnitt 10b entsprechen den jeweiligen Elementen der ersten Ausführungsform des Konnektors 1, es wird diesbezüglich auf die Ausführungen zur ersten Ausführungsform verwiesen. Das Durchstechelement 4' ist in seinem auf der Seite des ersten Anschlussbereiches 3a' liegenden Abschnitt, insbesondere im Endabschnitt 6a', entsprechend den Vorgaben des Male-Teils des Luer-Locks ausgebildet, der auf der Seite des zweiten Anschlussbereiches 3b liegende Teil entspricht dem Teil der ersten Ausführungsform des Konnektors 1. An den Konnektor 1' kann damit im ersten Anschlussbereich 3a' ein Behältnis mit einem Luer-Female-Teil angeschlossen werden. Das Female-Teil kann eine Membran aufweisen, die durch den Endabschnitt 6a' geöffnet werden kann. Alternativ kann das Female-Teil oder auch ohne Membran ausgebildet sein.

Der erfindungsgemäße Konnektor 1, 1' ist kostengünstig aus wenigen Teilen herstellbar, ermöglicht das sichere Verbinden zweier Behältnisse ohne die Gefahr, dass ein Behältnis beschädigt oder der Anwender verletzt wird, und erlaubt es, mit einer einfachen manuell ausführbaren Bewegung die Verbindung zwischen den beiden Behältnissen für die Überführung eines Wirkstoffes zu öffnen. Der Konnektor ist zu dem mit entsprechender Ausbildung des ersten Anschlussbereichs 3a, 3a' und des Verbindungsmittels 12 grundsätzlich für den Anschluss an beliebige Behältnisse geeignet, beispielsweise auch für den Anschluss an Spritzen oder Katheter.

## Patentansprüche

1. Konnektor (1, 1') für medizinischen Wirkstoff enthaltende Behälter (2), umfassend einen ersten Anschlussbereich (3a, 3a') für den Anschluss eines ersten Behälters (2), ein Durchstechelement (4, 4') und ein Anbrechelement (5), wobei das Durchstechelement (4, 4') einen ersten Endabschnitt (6a, 6a') und einen zweiten Endabschnitt (6b, 6b') und einen vom ersten Endabschnitt (6a, 6a') zum zweiten Endabschnitt (6b, 6b') verlaufenden Kanal (7) für den Transport eines medizinischen Wirkstoffs aufweist, wobei der erste Endabschnitt (6a, 6a') zum Durchstechen einer Membran (8) eines im ersten Anschlussbereich angeschlossenen ersten Behälters (2) ausgebildet ist, und am zweiten Endabschnitt (6b, 6b') das Anbrechelement (5) anbrechbar und/oder abbrechbar befestigt ist, wobei im befestigten Zustand das Anbrechelement (5) den Kanal (7) des Durchstechelements (4, 4') flüssigkeitsdicht verschließt.

2. Konnektor (1, 1') nach Anspruch 1, wobei das Durchstechelement (4, 4') und das Anbrechelement (5) einteilig ausgebildet sind.

3. Konnektor (1, 1') nach einem der vorhergehenden Ansprüche, wobei sich das Anbrechelement (5) hinterschnittsfrei an das Durchstechelement (4, 4') anschließt.

4. Konnektor (1, 1') nach einem der vorhergehenden Ansprüche, wobei das Durchstechelement (4, 4') und das Anbrechelement (5) aus Kunststoff, vorzugsweise aus Polypropylen, Polycarbonat und/oder einem Blend, vorzugsweise ein Blend aus Polypropylen und Styrol/Ethylen/Butylen/Styrol-Block-Copolymerisat sind.

5. Konnektor (1, 1') nach einem der vorhergehenden Ansprüche, zusätzlich umfassend ein Gehäuse (9, 9') mit einem den ersten Anschlussbereich (3) ausbildenden ersten Gehäuseabschnitt (10a, 10a') und einem Gehäuseboden (11, 11'), wobei das Durchstechelement (4, 4') durch den Gehäuseboden (11, 11') hindurchtritt.

6. Konnektor (1, 1') nach einem der vorhergehenden Ansprüche, zusätzlich aufweisend einen zweiten Anschlussbereich (3b) für den Anschluss eines zweiten Behälters.

7. Konnektor (1, 1') nach Anspruch 6 in Verbindung mit Anspruch 5, wobei das Gehäuse (9, 9') einen zweiten Gehäuseabschnitt (10b) aufweist, durch den der zweite Anschlussbereich (3b) ausgebildet wird.

8. Konnektor (1, 1') nach einem der Ansprüche 5 bis 7, wobei der erste Gehäuseabschnitt (10a, 10a'), der zweite Gehäuseabschnitt (10b) und/oder das Durchstechelement (4, 4') zumindest teilweise, vorzugsweise vollständig einteilig ausgebildet sind.

9. Konnektor (1, 1') nach einem der vorhergehenden Ansprüche, zusätzlich aufweisend ein zum Transport eines medizinischen Wirkstoffs ausgebildetes Verbindungsmittel (12) mit einem manuell elastisch deformierbaren Biegeabschnitt (13), wobei das Verbindungsmittel (12) den zweiten Endabschnitt (6b, 6b') umgibt und das Anbrechelement (5) sich zumindest teilweise in dem Biegeabschnitt (13) befindet, so dass durch Verformung des Biegeabschnitts (13) das Anbrechelement (5) anbrechbar und/oder abbrechbar ist.

10. Konnektor (1, 1') nach Anspruch 9 in Verbindung mit Anspruch 5, wobei das Verbindungsmittel (12) einen Verbindungsabschnitt (14) aufweist und das Verbindungsmittel (12) über den Verbindungsabschnitt (14) im zweiten Anschlussbereich (3b) an dem Gehäuse, gegebenenfalls an dem zweiten Gehäuseabschnitt (10b), befestigt ist.

11. Konnektor (1, 1') nach Anspruch 9 oder 10 in Verbindung mit Anspruch 5, zusätzlich aufweisend ein Dichtungsmittel (15), wobei das Dichtungsmittel (15) zwischen Verbindungsmittel (12) und Gehäuse (9, 9') angeordnet ist zum flüssigkeitsdichtem Abdichten des Verbindungsmittels (12) gegen das Gehäuse.

12. Konnektor (1, 1') nach einem der Ansprüche 9 bis 11, wobei das Verbindungsmittel (12) einteilig ist.

13. Konnektor (1, 1') nach einem der vorhergehenden Ansprüche, wobei gegebenenfalls das Gehäuse (9, 9') und/oder gegebenenfalls das Verbindungsmittel (12) aus Kunststoff sind, vorzugsweise aus Polypropylen, Polycarbonat und/oder einem Blend, vorzugsweise ein Blend aus Polypropylen und Styrol/Ethylen/Butylen/Styrol-Block-Copolymerisat, sind.

14. Konnektor (1, 1') nach einem der vorhergehenden Ansprüche, wobei gegebenenfalls das Gehäuse (9, 9') aus einem im Vergleich zum Verbindungsmittel (12) relativ steifen Material und das Verbindungsmittel (12) aus einem im Vergleich zum Gehäuse (9, 9') relativ flexiblen Material besteht.

15. Konnektor (1, 1') nach einem der vorhergehenden Ansprüche, wobei der erste Anschlussbereich (3a, 3a') zum Anschluss einer Glasflasche oder als Luer-Anschluss ausgebildet ist.

16. Konnektor (1, 1') nach einem der vorhergehenden Ansprüche, wobei das Verbindungsmittel (12) einen Schweißabschnitt (16) zum Anschweißen eines Beutels aufweist.
